# EUROPEAN PATENT APPLICATION

(11) **EP 3 315 942 A1**
(43) Date of publication of application: **02.05.2018**
(21) Application number: 17193182.7
(22) Date of filing: 26.09.2017
(51) Int. Cl.: G01N 21/07, G01N 21/59, B01L 3/00, G01N 21/75, G01N 35/00, G01N 21/84, G01N 21/17

(54) **TEST DEVICE, TEST SYSTEM, AND CONTROL METHOD OF THE TEST DEVICE**

(30) Priority: 27.10.2016 KR 20160140930
(71) Applicant: Samsung Electronics Co., Ltd., Gyeonggi-do 16677 (KR)
(72) Inventor: YEO, Yeong Bae, Seoul (KR); PARK, Sil, Gyeonggi-do (KR); BAE, Su Bong, Gyeonggi-do (KR)
(74) Representative: Grootscholten, Johannes A.M.

(57) **Abstract**

A test device (100), test system, and control method of the test device (100), which defines a light irradiating area in a reactor (20) to prevent a decrease in magnitude of a detected signal that may result due to scattering of light that has penetrated other area of the reactor (20) than an area (25) containing an object for detection and improve a dynamic range. A test device (100) may include a light source (130) configured to irradiate light; a reactor (20) configured to include at least one first area (25) to contain an object for detection; and a photo detector (150) configured to receive light that has been irradiated from the light source (130) and has passed the reactor (20) that contains the object for detection, wherein the light source (130) is configured to limitedly irradiate the light to the first area (25) of the reactor (20).

## Description

### BACKGROUND

### 1. Field

The present disclosure relates to a test device, test system, and control method of the test device for controlling light that has passed through a reactor to increase the magnitude of a signal detected from a test line.

### 2. Discussion of Related Art

As immunity tests, clinical chemistry tests, etc., are performed on samples from patients for in vitro diagnosis, the tests play important roles in diagnosis and treatment on the patent's disease and determination of its remission.

The in vitro diagnosis is often performed in test rooms or laboratories of hospitals, and may also be used for quick analysis of a sample in various fields, such as environmental monitoring, food inspection, medical diagnosis, etc. In order to perform the in vitro diagnosis in any setting, including the tests in the field, miniaturization of the in vitro diagnostic device is required.

In particular, when it comes to medical diagnosis, dependency on the point-of-care (POC) blood analyzer is ever increasing, and research and development for miniaturized POC blood analyzers that enable quick and accurate blood examination is actively underway all over the world.

As for the lab-on-a-chip or lap-on-a-disc based blood analyzer for the POC test, miniaturization and support for simultaneous tests on multiple items are very important factors. Accordingly, the lab-on-a-chip or lap-on-a-disc cartridge needs to be equipped with many detection chambers, and an optical detector is also capable of scanning and measuring the many detection chambers.

In the related art, a blood analyzer uses a back light unit that emits light over a wide area. This decreases the magnitude of a signal detected from a test line due to the scattering of light that has penetrated a reaction body without going through the reaction chamber of the reactor. This makes it difficult to detect a low-density bio marker from the reaction chamber.

### SUMMARY

Exemplary embodiments provide a test device, test system, and control method of the test device, which defines a light irradiating area in a reactor to prevent a decrease in magnitude of a detected signal that may result due to scattering of light that has penetrated areas of the reactor than an area containing an object for detection, thereby improving a dynamic range.

In accordance with an aspect of an exemplary embodiment, a test device may include an light source configured to emit light; a reactor including at least one first area configured to contain an object for detection; and a photo detector configured to receive the light that has been emitted by the light source and has passed through the reactor that contains the object for detection, wherein the light source is configured to limit light emitted to the first area of the reactor.

The test device may further include a controller configured to control the light source to emit light to be limited into the first area that contains the object for detection of the reactor.

The at least one first area may have the form of a strip.

The light source may include a linear light source to emit a line of light with a width less than a width of the first area of the reactor.

The controller may limit a width of light emission to be less than a width of the first area of the reactor.

The controller may control the photo detector to be moved to a position to receive the light that has passed through the first area.

The test device may further include a driver configured to rotate the reactor, wherein the controller is configured to control the driver to rotate the reactor and to position the reactor such that the light emitted by the light source passes through the first area.

In accordance with an aspect of another exemplary embodiment, a test system may include a light source configured to emit light; a photo detector configured to receive light that has been emitted from the light source and has passed through an object for detection; a reactor including at least one first area configured to contain the object for detection; and a mask located between the light source and the reactor to prevent light emitted from the light source from being emitted to at least one second area in the reactor.

The mask may include a slit configured to allow the light emitted by the light source to limitedly pass to the first area of the reactor.

The mask may include a slit with a width less than the width of the first area of the reactor.

The first area and the slit may have the form of a strip.

The light source may include a back light unit including at least one of a point light source, a linear light source, and an area light source.

The test system may further include a driver configured to rotate the reactor, wherein the driver is configured to rotate the reactor and to move the photo detector based on positions of the slit formed in the mask and the first area of the reactor.

In accordance with the other aspect of the present disclosure, a test device may include a light source configured to emit light; a photo detector configured to receive light that has been emitted from the light source and has passed through a reactor that contains an object for detection; and a controller configured to control the light source to emit light to be limited into a first area that contains the object for detection of the reactor, wherein the light source comprises: a back light unit configured to include at least one of a point light source, a linear light source, and an area light source; and a display panel configured to control an area to which the back light unit irradiates light.

The at least one first area may have the form of a strip.

The controller may control the display panel to be divided into the first area where the light source emits light and a second area to prevent irradiation of the light from the back light unit.

The display panel may further include a color filter configured to change a color of the light emitted by the back light unit, and the controller may control the light source to emit light of a selectable wavelength using the color filter.

The reactor may include identification information having data about the object for detection, and wherein the controller may control the light source to emit the light onto the identification information.

In accordance with an aspect of another exemplary embodiment, a method of analyzing an object for detection may include positioning a reactor containing the object for detection between a light source and a photo detector, the object for detection located in a first area of the reactor; controlling the light source to confine emission of light to a line of light having a width that is narrower than the first area of the reactor containing the object for detection; and operating the photo detector to receive the light that passes the object for detection.

The method may further include at least one of masking the light emitted from the light source and selectively activating components of the light source to output the line of the light.

The method may include use of a light source including at least one of a point light source, line light source, and area light source.

### BRIEF DESCRIPTION OF THE DRAWINGS

The above and/or other aspects will become more apparent by describing in detail exemplary embodiments with reference to the accompanying drawings, in which:
FIG. 1 is an exterior view of a test device, according to an exemplary embodiment;
FIG. 2 schematically shows a reactor, according to an exemplary embodiment;
FIG. 3 is a diagram for explaining the configuration of a test device with the reactor of FIG. 2 inserted thereto;
FIG. 4 is a control block diagram of a test device, according to an exemplary embodiment;
FIG. 5 is a diagram for explaining a problem of decrease in magnitude of a signal in a test line due to scattering of light irradiated by a light source according to conventional arrangements;
FIG. 6 is a front view for explaining optical measurement of a test device, according to an exemplary embodiment;
FIG. 7 is a plan view for explaining optical measurement of the test device of FIG. 6, according to an exemplary embodiment;
FIG. 8 is a perspective view for explaining optical measurement of a test device, according to an exemplary embodiment;
FIG. 9 is a front view for explaining optical measurement of a test device, according to another exemplary embodiment;
FIG. 10A is a plan view for explaining optical measurement of a test device, according to another exemplary embodiment; FIG. 10B is a modified embodiment of the test device of FIG. 10A;
FIG. 11 is a perspective view for explaining optical measurement of a test device, according to another exemplary embodiment;
FIG. 12 is a perspective view for explaining optical measurement of a test device, according to another exemplary embodiment;
FIGS. 13A, 13B, 13C and 13D are graphs for explaining effect of work of a test device according to an exemplary embodiment;
FIGS. 14A, 14B and 14C are graphs for explaining effect of work of a test device according to another exemplary embodiment;
FIG. 15 is a flowchart illustrating operation of a test device, according to an exemplary embodiment;
FIG. 16 is a flowchart illustrating operation of a test device, according to another exemplary embodiment;
FIG. 17 is a flowchart illustrating operation of a test device, according to another exemplary embodiment;
FIG. 18 is another exterior view of a test device, according to an exemplary embodiment;
FIG. 19 is an exterior view of a reactor to be inserted into the test device of FIG. 18; and
FIG. 20 is another exemplary example of an exterior view of a reactor.

### DETAILED DESCRIPTION OF EXEMPLARY EMBODIMENTS

Advantages, features, and apparatuses and methods for achieving them will be understood more clearly by the following description of exemplary embodiments with reference to the accompanying drawings. Exemplary embodiments of the disclosure may, however, be embodied in many different forms and should not be construed as limited to the embodiments set forth herein; rather, exemplary embodiments are provided so that this disclosure will be thorough and complete, and will fully convey the scope of the exemplary embodiments of the disclosure to those skilled in the art.

The term "include (or including)" or "comprise (or comprising)" is inclusive or open-ended and does not exclude additional, unrecited elements or method steps. Furthermore, the term 'unit' or 'module' refers to a software or hardware component, such as FPGA or ASIC programmable to perform one or more functions. However, the unit is not limited to software or hardware. The unit may be configured to be stored in an addressable storage medium, or to be executed one or more processors. For example, together with one or more processors or computers, the unit may include components, such as software components, object-oriented software components, class components and task components, processes, functions, attributes, procedures, subroutines, segments of program codes, drivers, firmware, microcodes, circuits, data, databases, data structures, tables, arrays, and variables. Functions served by components and units may be combined into a fewer number of components and units, or divided to be performed by a larger number of components and units.

The term 'user' as herein used may be a medical expert, e.g., a doctor, a nurse, a medical technologist, a medical image expert, etc., or a technician who fixes medical equipment, but is not limited thereto.

The term 'test device' as herein used is not limited to a device for the in vitro diagnosis, which is, however, taken as an example in embodiments.

A test system includes a reactor for containing a sample or a reagent, a test device for performing in vitro diagnosis once the reactor is inserted thereto, and other components. An exemplary embodiment of the test system for performing diagnosis after the reactor is inserted thereto will now be described.

A disc or a cartridge that may accommodate a sample or a reagent will be taken as an example of the reactor in an exemplary embodiment.

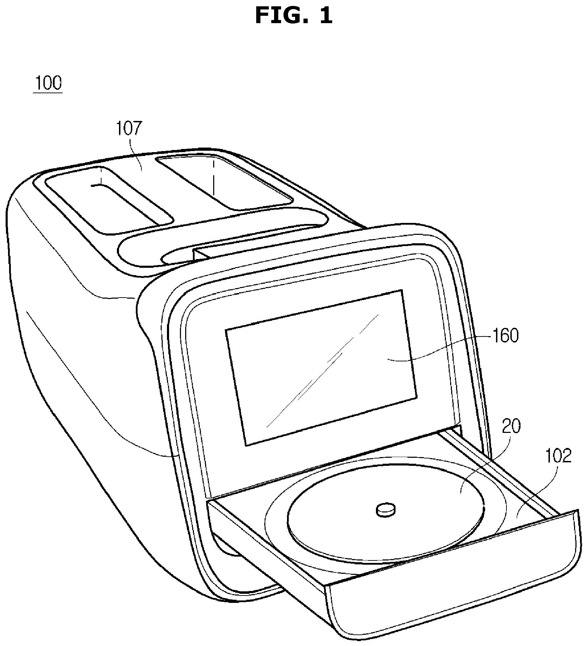

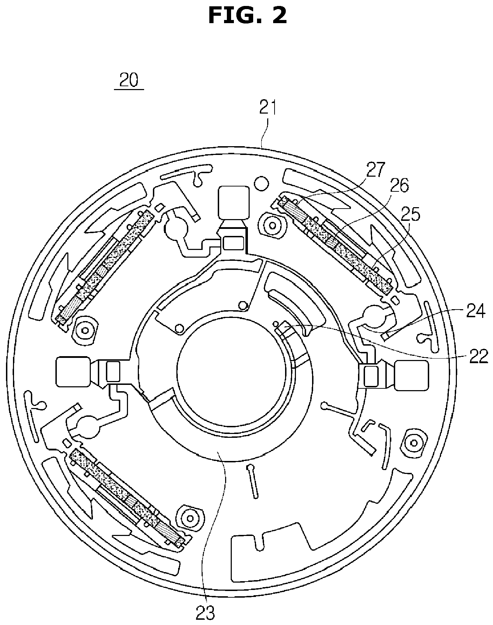

FIG. 1 is an exterior view of a test device, according to an exemplary embodiment, and FIG. 2 schematically shows a reactor, according to an exemplary embodiment.

A test device 100 is a miniaturized and automated device to be used in examining various kinds of samples, such as environmental samples, bio samples, food samples, etc. When the test device 100 is used for in vitro diagnosis, in particular, to examine a bio sample taken from a human body, Point of Care Testing (POCT) may be promptly performed by a user. Such user includes a patient, a doctor, a nurse, a medical technologist, etc., at a place where a patient is located. The location may be at a home, workplaces, outpatient clinics, hospital rooms, emergency rooms, operating rooms, intensive care units, as well as a test room.

The reactor, to which a sample is injected and in which there is a reaction between the sample and a reagent, may be of different types. The sample or the reagent is moved by capillary force in a cartridge type, or is moved by centrifugal force in a disc type. In a cuvette type, the sample or the reagent is not moved, but measured on the fly. Other types may also be used. The structure or configuration of the test device may vary by the type of the reactor. FIG. 1 illustrates an exemplary test device that uses rotation after the disc typed reactor is inserted thereto.

Referring to FIG. 1, the test device 100 may include a tray 102, on which a reactor 20 of the disc type may be seated. The seated reactor 20 may be inserted to the inside of a main body 107 of the test device 100 along with the tray 102.

Once the reactor 20 is inserted thereto, the test device 100 may rotate the reactor 20 according to a sequence determined by a type of the reactor 20, a type of a sample, or a test process, and measure a test result.

The test result may be output through a display 160 of the main body 107.

Referring to FIG. 2, the disc type reactor 20 may be in a circular shape.

Specifically, the reactor 20 may include a rotatable disc-shaped platform 21, at least one channel formed in the platform 21 to allow a fluid to flow therethrough, and at least one chamber for containing fluids.

The platform 21 may include multi-layered plates. Engraved structures that correspond to the chambers or the channels may be made on adjoining faces of the plates. The plates with engraved structures may then be joined together, thereby providing space and passages inside the platform 21. Such space and passages may form the aforementioned plurality of channels 24 and chambers 23, 25.

The plurality of channels 24 and chambers 23, 25 may include a sample chamber 23 for containing a sample such as blood in the central area of the platform 21, a reaction chamber 25 for containing a reagent such as a diluted solution to be mixed with the sample, and multiple channels 24 for connecting the chambers. One or more valves (not shown) control the flow of a fluid through the multiple channels.

The sample chamber 23 may contain a sample, e.g., blood taken from a patient through an injection port 22 to which the sample is injected. The sample chamber 23 may be divided into sections to contain predetermined amounts of the sample in the sections.

The reaction chamber 25 refers to a space where the sample reacts with the reagent. The reaction chamber 25 in accordance with an exemplary embodiment may have the form of a small and narrow rectangular strip, as shown in FIG. 2. The reaction chamber is not, however, limited to the strip shape, but may be implemented in various forms.

The channel 24 guides the sample contained in the sample chamber 23 to the reaction chamber 25. While the disc type reactor 20 is rotated, the centrifugal force makes the sample in the sample chamber 23 flow along the channel 24 to the outer side of the reactor 20, mixed with other reagents, and flow into the reaction chamber 25.

In this regard, if an amount of the contained sample is small, the sample may flow into a single reaction chamber 25 on the platform 21. On the contrary, if the amount of the sample is large, the sample may flow into a plurality of reaction chambers 25 as shown in FIG. 2.

In the meantime, if the reactor 20 is rotated at high speed, centrifugation of the sample, e.g., blood, may occur. For example, after the centrifugation, the blood is divided into serum and corpuscles. The separated serum may be moved to one or more of reaction chambers 25 along the corresponding channel 24.

Specifically, if the rotation of the reactor 20 is stopped, the serum that went through the channel 24 is absorbed by the reaction chamber 25. For example, the reaction chamber 25 sucks in the separated serum with an absorptive pad (not shown). The serum undergoes reactions while passing a control line 26 and a test line 27 in sequence.

The reactor 20 may contain a reagent. The reagent may be contained in a manner of being applied within the reaction chamber 25 and then dried. As described above, the serum reacts with the contained reagent while being absorbed by the reaction chamber 25.

Hereinafter, a sample that reacts with a reagent will be referred to as an object for detection.

The platform 21 may be made of a plastic material such as acrylyl, poly(dimethylacrylamide) (PDMA), or polymethylmethacrylate (PMMA), which is easy to be molded and has a biologically inert surface. The platform 21 is not, however, limited thereto, but may be made of any of biologically stable, optically transparent, and mechanically processable materials.

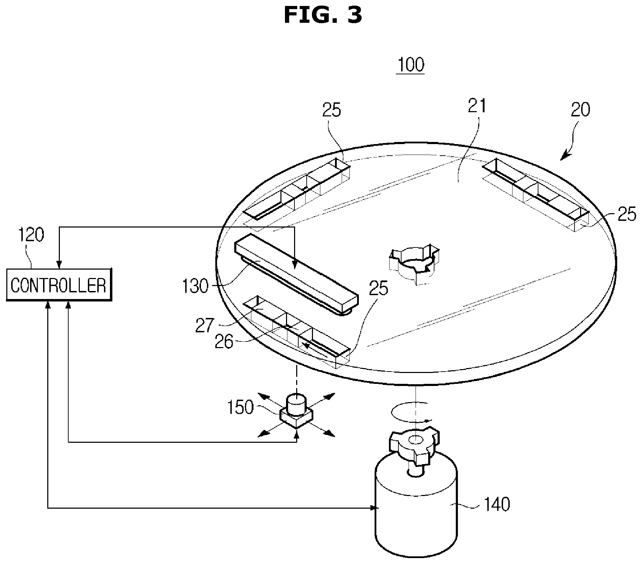

Fig. 3 explains the test device with the reactor of Fig. 2 inserted.

Referring to Fig. 3, the reactor 20 in an exemplary embodiment is optically transparent to pass the light irradiated from a light emitter 130 which includes a light source. Component structures including channels and the reaction chamber 25, which contains the object for detection, may also have properties of optical transparency.

The light irradiated from the light source 130 may penetrate the object for detection contained in the reaction chamber 25 and thus result in a color of a certain wavelength. A photo detector 150 captures the light that has passed the object for detection and sends the result to a controller 120, which in turn analyzes the object for detection based on the captured result.

In the past, images with uniform brightness were acquired because the light source irradiated light onto a wide area. With the light irradiated onto the wide area, objects for detection placed in the reaction chambers located at various positions in the reactor 20 were examined. In the conventional case, however, the widely irradiated light also passed other optically transmissive elements in addition to the reaction chamber 25. Due to the scattering of light, the magnitude of a signal resulting from any reaction at the position of the test line 27 within the reaction chamber 25 deteriorates.

Referring to FIG. 3, the disc type test device 100 may include the rotatable reactor 20, a driver 140 for rotating the reactor 20, the light source 130 for irradiating light to an object for detection contained in the reaction chamber 25 of the reactor 20, the photo detector 150 for detecting light that has passed the object for detection, and the controller 120 for controlling the enumerated components.

First, the driver 140 rotates the reactor 20 using a spindle motor. The driver 140 may create centrifugal force to transfer a sample in the reactor 20 or move various structures in the reactor 20 to desired positions by rotating and stopping rotation of the reactors. The driver provides rotation and stop operations upon reception of signals output from the controller 120.

The driver 140 may also include a motor driver to control the angular position of the reactor 20. For example, the motor driver may use a step motor or a direct current (DC) motor.

The light source 130 irradiates light to the reaction chamber 25 that has absorbed the sample.

For example, the light source 130 and the photo detector 150 may be arranged on a same side of the reactor. The light source 130 may preferably be arranged to face the photo detector 150 as shown in FIG. 3. Although FIG. 3 shows that the light source 130 and the photo detector 150 are located on the upper and lower sides, respectively, with the reactor 20 between them, the positions may be interchangeable. The light source 130 may control an amount of light to be irradiated under the control of the controller 120.

The reaction chamber 25 in accordance with an exemplary embodiment may have the form of a narrow and elongated strip, as shown in FIG. 3. As described above , in the case of the conventional light source that irradiates light with an area corresponding to the entire area of the reactor 20, scattering of the light may drop the sensitivity of a measuring signal of the reaction chamber 25.

To solve this problem, the test device 100 in accordance with an exemplary embodiment may have a light source of the light source 130 based on the shape of the reaction chamber 25. For example, the light source 130 may be implemented with a linear light source, as shown in FIG. 3.

Although the linear light source may refer to a light source that emits light of one or more narrow spectra rather than continuous spectrum, the linear light irradiated by the light source 130 in an exemplary embodiment refers to a light source that irradiates linear rays whose emission surface has the form of a spatially narrow line.

The photo detector 150 detects an object for detection by receiving the light that has been irradiated by the light source 130 and has passed or has been reflected from the object for detection.

In the test device 100 according to an exemplary embodiment, a photo detector 150 is installed on a radially movable structure to detect a plurality of objects for detection placed in the reactor 20. The positional movement is shown by the directional arrows in Fig. 3. The photo detector 150 may be moved under the control of the controller 120 to a position at which the magnitude of a detected signal increases.

Although the single photo detector 150 is shown in FIG. 3, an exemplary embodiment may be implemented with plural photo detectors. Furthermore, it is not necessary for the photo detector 150 to be movable, but may be fixedly arranged.

Once the photo detector 150 obtains an image of an object for detection by receiving the light that has passed or has been reflected from the object for detection, the controller 120 may determine whether the object for detection exists in the position where the reaction has occurred and detect a concentration of the object for detection.

The reaction chamber 25 in an exemplary embodiment may contain a reagent and include the control line 26 and the test line 27.

Specifically, once a sample is absorbed by the reaction chamber 25, it moves from one end to the other end. The sample reacts with the reagent, and then becomes the object for detection.

The sample that has reacted with the reagent reaches the control line 26 first. At this time, if the photo detector 150 captures an image of the sample reacting with the test line 27, the controller 120 may determine that the sample has been normally contained and absorbed in the reaction chamber 25. Subsequently, if the sample continues to be absorbed and reaches the test line 27, the controller 120 makes a diagnosis from the image captured by the photo detector 150 based on the concentration of the object for detection.

The controller 120 is a processor for controlling general operation of the test device 100.

The controller 120 may be implemented with a memory (not shown) storing an algorithm to control operation of the components of the test device 100 or data about a program that implements the algorithm, and a processor (not shown) carrying out the aforementioned operation using the data stored in the memory. The memory and the processor may be implemented in separate chips. Alternatively, the memory and the processor may be implemented in a single chip.

For example, the controller 120 controls the light source 130 to irradiate light, controls the driver 140 to rotate the reactor 20, and analyzes an image captured by the photo detector 150 by controlling operation and movement of the photo detector 150.

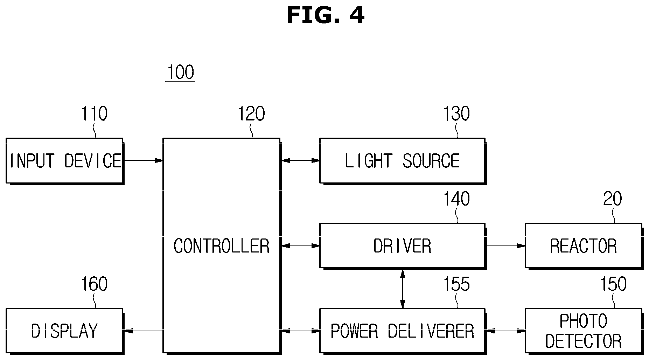

In addition, the controller 120 may control a valve inside the reactor 20 or control other operations to dehydrate the sample, store and output detection results. In addition to the operations as described above, the controller 120 may control overall operation of the test device 100. Operation of the controller 120 will now be described in detail in connection with FIG. 4.

FIG. 4 is a control block diagram of a test device, according to an exemplary embodiment.

Referring to FIG. 4, the test device 100 may include an input unit 110 for receiving the user's instructions, the controller 120 for controlling general operation of the test device 100, the light source 130 for irradiating light to the reactor 20, the photo detector 150 for receiving light that has passed the reactor 20, a power deliverer 155 for delivering power to the photo detector 150, and a display 160 for receiving an image output by the photo detector 150 to output a diagnosis result determined by the controller 120.

The input unit 110 may include many different buttons or switches, a pedal, a keyboard, a mouse, a track ball, various levers, a handle, a stick, or other hardware devices for receiving user input.

For example, the user may settle the reactor 20 on the tray 102 and then input a command through the input unit 110. After receiving the input command, the input unit 110 may send the command to the controller 120, which may in turn move the tray 102 for the reactor 20 to be inserted into the main body 107.

The input unit 110 may also include a Graphical User Interface (GUI), e.g., a software device, such as a touch pad for the user input. The touch pad may be implemented with a Touch Screen Panel (TSP), thus forming an interlayer structure with the display 160 as will be described later.

The display 160 may also be used for the input unit 110 if implemented with the TSP that forms the interlayer structure with the touch pad.

The light source 130 may be implemented by a point light source, a linear light source, or an area light source, which is able to irradiate light to the reactor 20. For example, a back light unit may be used for the light source 130.

Furthermore, the light source 130 may be a light source that flickers at a certain frequency, which may be implemented by a display panel including a semiconductor light emitting device, such as a Liquid Crystal Display (LCD), a Light Emitting Diode (LED), or an Organic Light Emitting Diode (OLED), or by a gas discharge lamp such as a halogen lamp or a xenon lamp.

Specifically, for example, the light source 130 may be implemented with a linear light source to be tailored to the shape of the reaction chamber 25. In another example, the light source 130 may be implemented with a point light source or an area light source, but may be divided to selectively irradiate light under the control of the controller 120 and operate to minimize scattering of the light passing the reactor 20. This will be described later in more detail with reference to accompanying drawings.

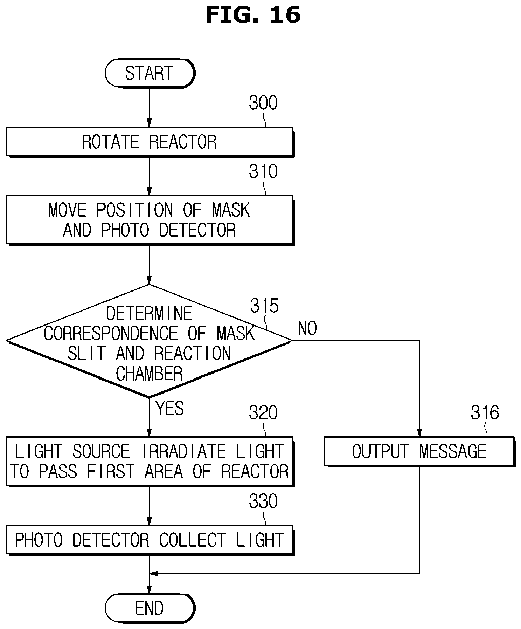

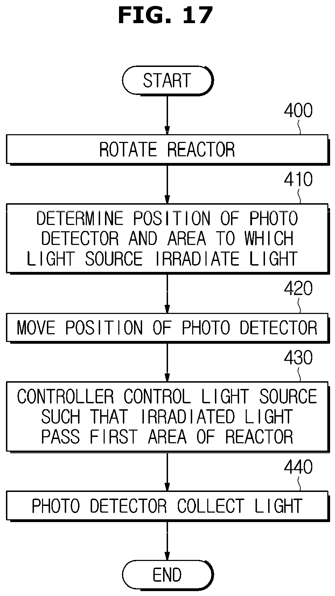

The reactor 20 may be a disc type reactor 20 as described above in connection with FIG. 2, or a cartridge type reactor 10 as will be described in connection with FIGS. 16 and 17. In the following description, the disc type reactor 20 will be taken as an example for convenience of explanation.

The disc type reactor 20 may be rotated by rotary power delivered by the driver 140. The reactor 20 uses the rotary power to move the sample to the reaction chamber 25 or to centrifuge the sample.

The driver 140 may also include a motor driver to control the angular position of the reactor 20. For example, the motor driver may use a step motor or a DC motor.

The rotary power generated by the driver 140 may be used not only to rotate the reactor 20 but also to move the position of the photo detector 150. Specifically, the rotary power generated by the driver 140 may be transformed by the power deliverer 155 into linear motion and used to control the position of the photo detector 150.

For example, upon receiving a command to start diagnosis from the user through the input unit 110, the controller 120 controls the driver 140 to rotate the reactor 20. If the rotation of the reactor 20 is stopped, the controller 120 may use the power deliverer 155 to move the photo detector 150 to a proper position to receive light that has passed the reaction chamber 25.

The photo detector 150 detects the light that has been irradiated from the light source 130 and has penetrated or has been reflected from an object for detection contained in the reaction chamber 25 of the reactor 20. Moreover, the photo detector 150 may generate an electric signal according to color or intensity of the light that has passed the object for detection. The electric signal is sent to the controller 120 and used to analyze or diagnose the object for detection.

For example, the photo detector 150 may be placed to face the light source 130. The test device 100 in an exemplary embodiment may control an area, into which the light source 130 irradiates light or an area for the reaction chamber 25 to pass light in order to minimize scattering of the light. Accordingly, the scattered light of the light source 130 may be shielded from the reaction chamber 25 by adjusting a capturing angle (or viewing angle). This will be described later in more detail with reference to accompanying drawings.

Meanwhile, the photo detector 150 may include a depletion layer photo diode, an avalanche photo diode, or a photomultiplier tube. Furthermore, the photo detector 150 may also be implemented with a complementary metal-oxide semiconductor (CMOS) image sensor or a charge coupled device (CCD) image sensor.

The display 160 may display results of examination or analysis performed by the test device 100. As described above, as the reactor 20 may include multiple chambers 25, multiple test items may be detected from a single reactor 20, and the display 160 may display the detection results of the multiple test items. The display 160 may also provide the user with various information relating to the test device 100, e.g., the position of the photo detector 150 or detection accuracy of the photo detector 150 in receiving light.

The display 160 may be implemented by a presentation means, such as LCD, LEDs, OLEDs, Active Matrix Organic Light Emitting Diodes (AMOLEDs), flexible display, three dimensional (3D) display, etc. Furthermore, as described above, the display 160 may include a touch screen that receives touch inputs from the user.

In addition, the test device 100 may include other various components, such as various sensors to measure the temperature of the reactor 20, a heater (not shown) to control the temperature of the reactor 20, a communication unit (not shown) for the test device 100 to transmit or receive data to or from an external server or to store data related to control the test device 100 in the external server, etc., without being limited thereto.

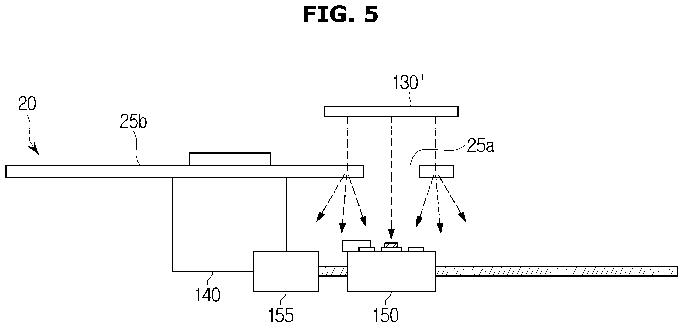

FIG. 5 is a diagram for explaining a problem of decrease in magnitude of a signal in a test line due to scattering of light that has been irradiated by a light source in a conventional arrangement. The diagram of FIG. 5 is an internal configuration of the test device 100 viewed from the front.

Referring to FIG. 5, in a conventional test device, the emitter 130' is formed to have a large area to irradiate light uniformly to the reactor 20. In the case of irradiating light into a large area, the photo detector 150 may obtain a uniformly bright image.

As shown in FIG. 5, the reactor 20 is formed of an optically transmissive material. This makes the light irradiated from the emitter 130' penetrate not only the reaction chamber 25a in the reactor 20 but also the other area of the reactor 20.

In this regard, an area of the platform 21 of the reactor 20, in which the reaction chamber is denoted a first area 25a, and the other area of the platform 21 than the reaction chamber is denoted a second area 25b.

The light that has penetrated the second area 25b may scatter, as shown in FIG. 5.

In an exemplary embodiment, the platform 21 of the reactor 20 may have a structure in which three plates are joined.

The three plates are divided into top, bottom, and middle plates, and the top and bottom plates are made of films. The films used to form the top and bottom plates may be one selected from among polyethylene films, such as very low density polyethylene (VLDPE), linear low density polyethylene (LLDPE), low density polyethylene (LDPE), medium density polyethylene (MDPE), high density polyethylene (HDPE), etc., polyvinyl chloride (PVC) films, polyvinyl alcohol (PVA) films, polystyrene films, and polyethylene terephthalate (PET) films.

The middle plate may be formed of a porous sheet, such as cellulose to serve as a vent in itself, and the porous sheet may be made of a hydrophobic material or a hydrophobic process is applied on the porous sheet in order not to influence the movement of the sample.

The platform 21 may be formed in such a way that the three plates as joined may optically bend the irradiated light. In other words, the second area 25b of the reactor 20 is also optically transparent to pass the light irradiated from the light source 130, but may scatter the light.

For in vitro diagnosis, the ability to detect a low-density bio marker is an important performance index. When it comes to a cardiovascular marker of blood in particular, such as Cardiac Troponins (Troponin I (TnI); Troponin T (TNT)), sensitivity of detection is important to diagnose a disease in its early stage.

However, as shown in FIG. 5, if the light that has penetrated the second area 25b scatters and is collected by the photo detector 150, there is difficulty in detecting the low-density bio marker, which has passed the first area 25a corresponding to the reaction chamber 25. This is because the magnitude of a signal that has passed the first area 25a may be reduced as the scattered light is received.

Accordingly, the test device 100 in accordance with an exemplary embodiment increases efficiency in detection of the low-density bio marker by minimizing the scattering of light in the second area 25b that is received by the light detector.

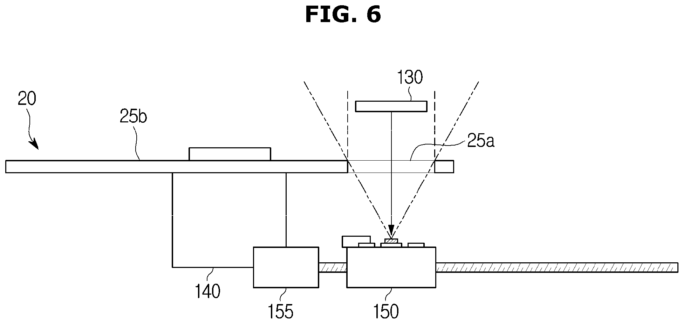

FIG. 6 is a front view for explaining optical measurement of a test device, according to an exemplary embodiment.

Referring to FIG. 6, the light source 130 and the photo detector 150 may be arranged in positions opposite the reactor 20, specifically, the first area 25a with certain gaps.

The reactor 20 is rotated according to the rotary power delivered by the driver 140 and then moves the sample into the first area 25a. The photo detector 150 is moved by the power deliverer 155 to a position corresponding to the first area 25a based on the rotated position of the reactor 20.

Subsequently, the light source 130 irradiates light, and the light passes an object for detection contained in the first area 25a and is then received by the photo detector 150.

At this time, the light source 130 may irradiate light with a light source having a width less than the width of the first area 25a by a predetermined value, as shown in FIG. 6.

As the light source 130 irradiates light into the width less than a predetermined value, the light source 130 is hidden by the first area 25a within a capturing angle of the photo detector 150. In other words, the light source 130 irradiates light confined within a width of the first area 25a, and the photo detector 150 has a capturing angle that corresponds more precisely to the first area 25a.

The test device 100 in accordance with an exemplary embodiment may limit the width of the light source of the light source 130 such that the irradiated light passes only the first area 25a, and thus maximize light measurement ability of the photo detector 150.

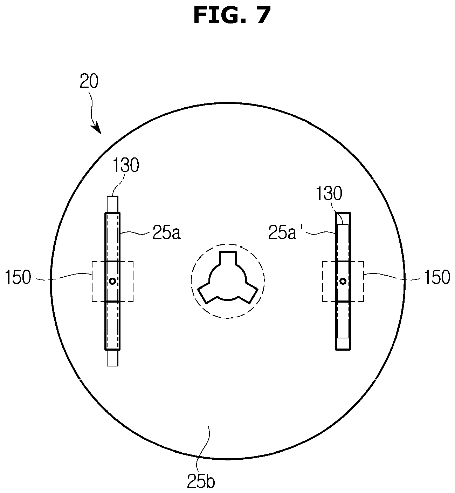

FIG. 7 is a plan view for explaining optical measurement of the test device of FIG. 6, according to an exemplary embodiment.

Referring to FIG. 7, a two dimensional (2D) area of the reaction chamber 25, which contains an object for detection, corresponds to the first area 25a and 25a' in the reactor 20. Further, the other area than the first area 25a and 25a' in the reactor 20 corresponds to the second area 25b.

FIG. 7 shows the inside of the test device 100 viewed from the top of the main body 107. Specifically, the test device 100 in an exemplary embodiment may be configured in the form in which the light source 130, the first area 25a, and the photo detector 150 are arranged in order from the top, as shown in FIG. 7.

Taking a look at the first area 25a shown on the left of the reactor 20, the width of the light source of the light source 130 may be narrower than the width of the first area 25a. For example, the horizontal length of the light source 130 is less than that of the first area 25a. This may prevent scattering of the light irradiated by the light source 130 in the second area 25b, enabling the light to pass the first area 25a and be collected correctly, e.g., with minimized scattering, by the photo detector 150. For the sake of clarity, the sample chamber and delivery channel are not illustrated for these discussions.

In an exemplary embodiment, not only the horizontal length of the light source 130 is limited in the test device 100. For example, taking a look at the first area 25a' shown on the right of the reactor 20 shows that the vertical length of the light source 130 may be equal to or shorter than the length of the first area 25a' as well. The total area of the light source of the light source 130 is also less than the area of the first area 25a'.

In the meantime, the total length of the light source of the light source 130 in the first area 25a shown on the left in FIG. 7 may be larger than the length of the first area 25a.

Even if the width of the light source 130 is limited in accordance with an exemplary embodiment, it is not necessary for the length of the light source 130 to be less than a length of the first area 25a. The area illuminated by the light source may be larger than 25a, but the irradiated area has a width smaller than the first area 25a. In other words, as the width of the light source of the light source 130 is limited, it is sufficient for the photo detector 150 to collect the light that has passed the first area 25a, without being limited thereto.

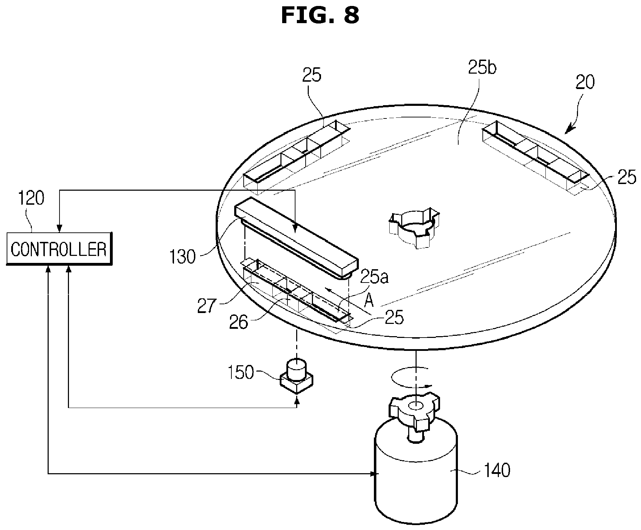

FIG. 8 is a perspective view for explaining optical measurement of a test device, according to an exemplary embodiment.

Referring to FIG. 8, the test device 100 may include the driver 140 for generating rotary power under the control of the controller 120, the reactor 20 that is rotated by the power of the driver 140, the reaction chamber 25 arranged on the platform 21 of the reactor 20, the light source 130 for irradiating light under the control of the controller 120, and the photo detector 150 for receiving light that has passed the reaction chamber 25.

As described above in connection with FIG. 2, the reactor 20 that may be rotated by the driver 140 may distribute a sample to the reaction chamber 25 through the channel 24. For example, the reaction chamber 25 absorbs the delivered sample, which is moved in the direction of an arrow A shown in FIG. 8.

The sample moving in the reaction chamber 25 sequentially reacts with reagents in the movement path. The sample that has reacted with the reagent, e.g., the object for detection, may change the light irradiated by the light source 130 to have a particular wavelength, and the photo detector 150 may collect the light that has passed the object for detection.

Specifically, the photo detector 150 may receive light that has passed the object for detection moving along the control line 26. For example, if the photo detector 150 takes an image of the object for detection moving along the control line 26, the controller 120 may determine that the sample has normally arrived at the reaction chamber 25.

Subsequently, the sample keeps moving and the object for detection passes the test line 27. The photo detector 150 captures an image of the light that has penetrated the object for detection located in the test line 27 and sends the captured results to the controller 120. The controller 120 makes a diagnosis based on the captured result.

Meanwhile, to increase measurement sensitivity of the test device 100 and minimize the effects from the scattering of light, the width of the light source 130 is limited. Specifically, the width of the first area 25a corresponding to the 2D area of the reaction chamber 25 may be larger than the width of the light source 130, as shown in FIG. 8.

In this case, the photo detector 150 capturing angle is confined to light of the first area 25a within the capturing angle, so the scattered light of light source 130 is not shown and the photo detector 150 collects only the light that has passed the object for detection.

In FIG. 8, the light source 130 and the photo detector 150 are shown to operate in one of a plurality of reaction chambers 25 located in the reactor 20. This is, however, merely for convenience of explanation, and the test device 100 in accordance with the present disclosure may include a plurality of light sources 130 for capturing images of objects for detection in multiple reaction chambers 25 in the reactor 20.

Furthermore, the photo detector 150 may be moved to receive the light that has passed the objects for detection in the plurality of reaction chambers 25, or the test device may include multiple photo detectors.

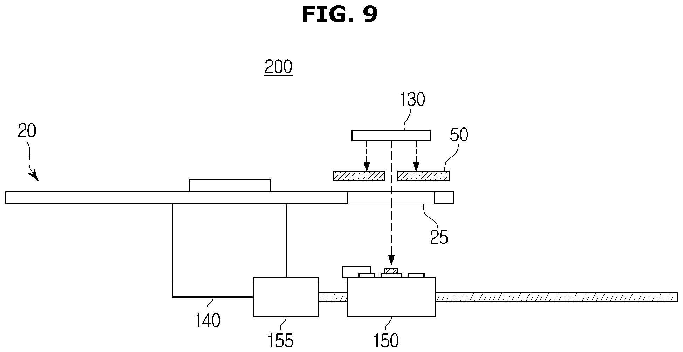

FIG. 9 is a front view for explaining optical measurement of a test device, according to another exemplary embodiment.

Referring to FIG. 9, the test device 100 may include the light source 130 for irradiating light, the reactor 20 for containing an object for detection, the driver 140 for rotating the reactor 20, the photo detector 150 for receiving light that has passed the object for detection, and the power deliverer 155 for moving the photo detector 150. The test device 100 may further include a mask 50 to limit the irradiated light to pass only the object for detection.

Configurations of the test device 200 that overlap with what are described above will be omitted.

Unlike in FIG. 6, the light source 130 irradiates light to a large area of the reactor 20. If there were no mask 50, the light that has passed an area other than the optically transmissive reaction chamber 25 in the reactor 20 would scatter and be received by the photo detector 150.

To solve this problem, the mask 50 may limit the light irradiated by the light source 130 to pass only the reaction chamber 25. Furthermore, the mask 50 may be arranged between the light source 130 and the reactor 20, and it is preferable to press the mask 50 against the reactor 20.

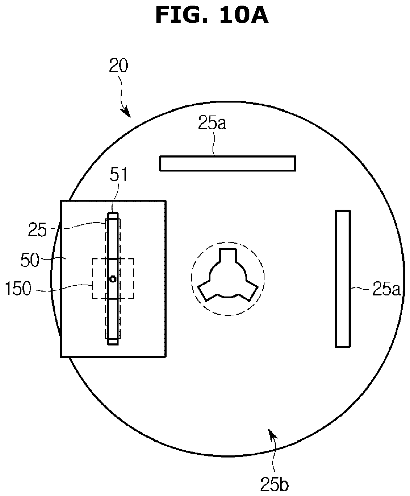

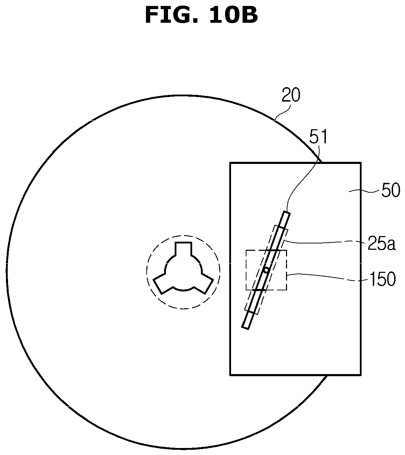

FIG. 10A is a plan view for explaining optical measurement of a test device, according to another exemplary embodiment. FIG. 10B is a modified exemplary embodiment of the test device of FIG. 10A. The embodiment will be described in connection with FIGS. 10A and 10B together to avoid overlapping explanation.

When the inside of the test device 100 is viewed from above, configurations of the reactor 20 may be shown as in FIG. 10A.

The area where the reaction chamber is arranged on the top plate of the reactor 20, e.g., may include several first areas 25a. The mask 50 may be placed over one of the plurality of first areas 25a.

It is not necessary for the mask 50 to be located in a single first area 25a. A plurality of masks 50 may be located to correspond to positions of a plurality of first areas 25a.

Referring to FIG. 10A, the central part of the mask 50 in an exemplary embodiment may have a strip-shaped slit or groove 51. The width of the central groove 51 of the mask 50 may be less than the width of the first area 25a.

In other words, the light from light source 130 is confined to the first area 25a and light passed through is within the capturing angle of the photo detector 150 located below the reactor 20. This may make the light irradiated by the light source 130 pass only the first area 25a and reach the photo detector 150, thereby improving sensitivity of detection by minimizing the scattering of light.

The width of the central slit 51 of the mask 50 shown in FIG. 10A may vary. For example, although only the width of the center slit 51 is less than the width of the first area 25a in FIG. 10A, the length of the center slit 51 may be equal to or less than the length of the first to provide a lighting profile similar to that of the right hand side area 25a as shown in FIG. 7.

FIG. 10B is a modification of the test device 100. Specifically, the first area corresponding to the reaction chamber in the reactor 20 is not necessarily limited to the shape as shown in FIG. 10A.

As shown in FIG. 10B, the first area 25a may be oriented or shaped differently and placed in the reactor 20, and the mask 50 may be changed according to the size and shape of the first area 25a.

The slit 51 of the mask 50 need not be placed in the center of the mask 50, but may be located on a side as shown in FIG. 10B. Furthermore, the slit 51 may not be necessarily strip-shaped. In other words, the mask 50 may have a slit with any size that fits the shape of the first area 25a to limit the light irradiated by the light source 130.

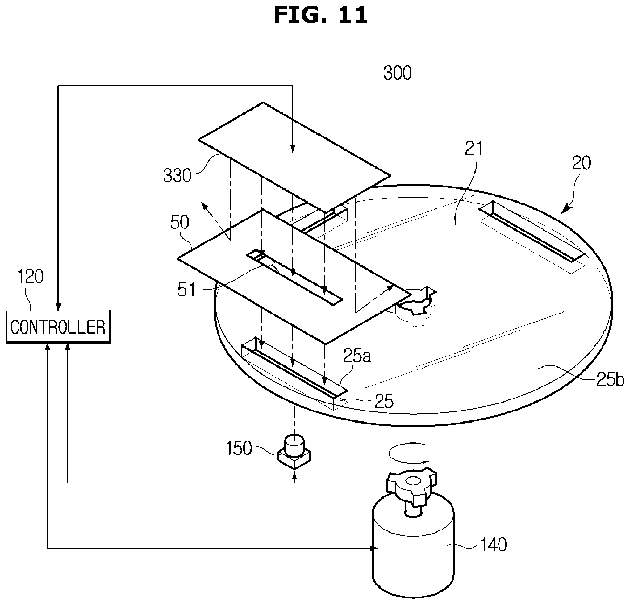

FIG. 11 is a perspective view for explaining optical measurement of a test device, according to another exemplary embodiment.

The test device 300 may include the light source 330 for irradiating light, the mask 50 for limiting the irradiated light not to pass the entire reactor 20, the reaction chamber 25 located in the reactor 20 for containing an object for detection, the photo detector 150 for receiving the light that has passed the reaction chamber 25, the driver 140 for rotating the reactor 20, and the controller 120 for controlling the enumerated components.

Unlike the light source of the test device 100 shown in FIG. 8, the light source 330 here may include an area light source. Specifically, the light source 330 of FIG. 11 may be provided as a back light unit that does not irradiate linear light to fit the shape of the first area 25a corresponding to the reaction chamber 25 but irradiate a wide area of light.

The light source 330 may irradiate light into a wide area of the reactor 20, e.g., even over the second area 25b as well as the first area 25a. Accordingly, to improve efficiency of detection, the test device 100 further includes the mask 50.

The central slit 51 of mask 50 confines the light emitted by light source 330. The confined light reaches the reaction chamber 25 of the reactor 20, and then penetrates the object for detection contained in the reaction chamber 25 and is collected by the photo detector 150. The photo detector 150 may send the detected image to the controller 120, which in turn makes a diagnosis based on the detected image.

In FIG. 11, the mask 50 is located at a certain distance from the reactor 20. This is for the convenience of explanation and to distinguish the components from each other. It is preferable for the mask 50 to be positioned pressed against the top face of the reactor 20.

Furthermore, the mask 50 is not limited to having the rectangular shape as shown in FIG. 11, but may have the same shape as the reactor 20, e.g., the circular shape. In other words, the mask 50 may be formed in any shape and size as long as it has the slit 51 formed to have the light irradiated by the light source 330 pass the first area 25a only.

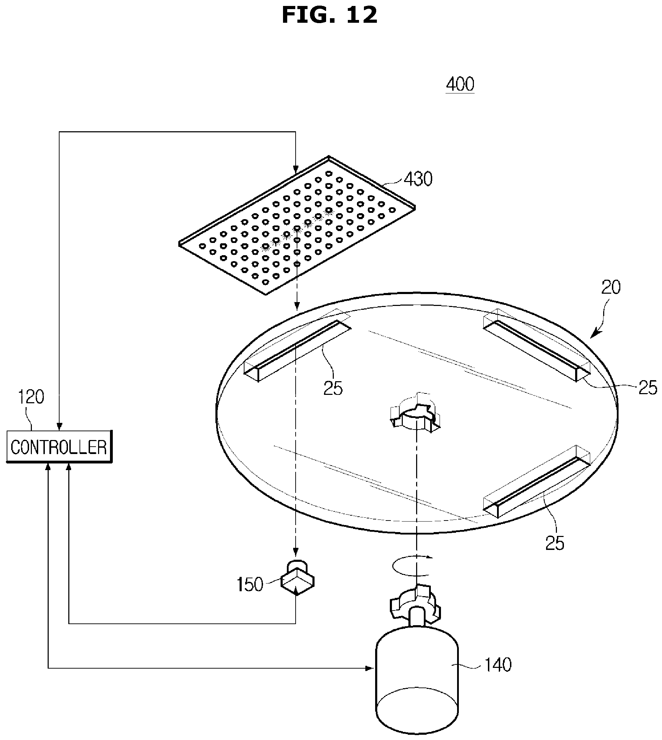

FIG. 12 is a perspective view for explaining optical measurement of a test device, according to another exemplary embodiment. Specifically, unlike FIG. 11, FIG. 12 shows a perspective view of the test device 400 viewed from below.

Referring to FIG. 12, the test device 400 may include the light source 430 for adjusting the position and intensity of light to be irradiated, the driver 140 for providing rotary power, the reactor 20 that is rotated by the rotary power delivered from the driver 140, the reaction chamber 25 formed on the platform 21 of the reactor 20 for containing the object for detection, the photo detector 150 for receiving light that has passed the object for detection, and the controller 120 for controlling the enumerated components.

Among the components included in the test device 400, those already described above will be omitted in the following description.

In the test device 400, the light source 430 may include a display panel.

Specifically, the light source 430 may include a light emitting display panel that emits light for itself, and a non-light emitting display panel that requires an extra light source. The light emitting display panels include Cathode Ray Tube (CRT) panels, Electro Luminescence (EL) panels, Organic Light Emitting Diode (OLED) panels, Vacuum Fluorescence Display (VFD) panels, Field Emission Display (FED) panels, Plasma Display Panels (PDPs), etc., and the non-light emitting display panels include Liquid Crystal Display (LCD) panels.

The LCD panel may further include a back light unit for emitting light from the rear side of the LCD panel, and the light emitted from the back light unit may be colored according to color filters included in the LCD panel.

The display panel may adjust the light emitting area. For example, in the case of the light emitting display panel, the light emitting area may be adjusted by selectively controlling light sources to which power is supplied for light emission to the reactor. Furthermore, the non-light emitting display panel may selectively control the light emitting area by controlling the back light unit or a substrate having a Thin Film Transistor (TFT) array thereon to control irradiation of light.

In FIG. 12, schematic operation of the light source 430 controlling a region of light sources to irradiate light to the reactor 20 is shown. The light source 430 of FIG. 12 turns on some of many point light sources to irradiate light only to the first area 25a.

By selectively turning on some light sources, the light source 430 may irradiate light to the reaction chamber corresponding to the first area 25a as described above, and the photo detector 150 may collect light that has passed the object for detection under a minimized influence of the scattering of light in other area of the reactor 20, thereby having good sensitivity of detection.

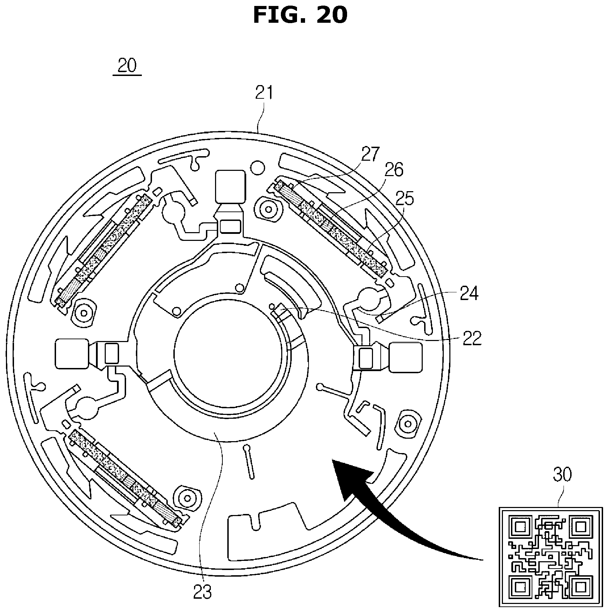

The light source 430 including the display panel may selectively turn on the light sources for taking an image of another area of the reactor 20 that includes identification information, for example, as will be described below with reference to FIG. 20. This allows for capturing data from an optimum area and increased power efficiency.

The display panel as a light source may also selectively control wavelengths of the light sources for irradiation by controlling color filters.

Specifically, the color filters refer to filters including dyes or pigments that absorb or transmit a particular range of wavelengths. For example, the color filters may include a blue color filter that transmits blue color but absorbs other colors than the blue color; a green color filter that transmits green color but absorbs other colors than the green color; and a red color filter that transmits red color but absorbs other colors than the red color.

In an exemplary embodiment, it is also possible for the light source 430 to selectively irradiate light of a particular wavelength by controlling the color filters.

If the reaction chamber 25 uses gold nanoparticles, sensitivity to the light of green wavelength may preferably be used. In this case, the controller 120 may control the light source 430 for the display panel to irradiate the light of green wavelength.

In FIG. 12, operation of the light source 430 irradiating light to just one of the plurality of reaction chambers 25 arranged in the reactor 20 is shown. This is, however, merely for the convenience of explanation, and the light source 430 may include a display panel that irradiates light into the entire area of the reactor 20. Furthermore, the light source 430 may irradiate light only to each of the reaction chambers 25 under the control of the controller 120, or selectively irradiate light to a position requiring irradiation of the light.

Selective light irradiation of the light source 430 may adjust a region in which light sources are turned on regardless of the shape or position of the reaction chamber 25. If a different reactor 20' is inserted to the test device 400, having a different location or shape of the reaction chamber, the test device 400 may dynamically tune a light irradiation area via light source 430.

The display panel of the light source 430 may include other components than the aforementioned components, without being limited thereto.

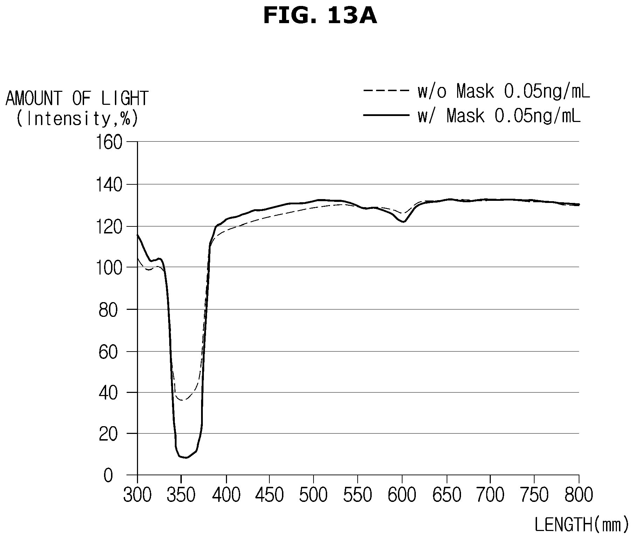

FIGS. 13A to 13D are graphs for explaining effects of test devices according to exemplary embodiments. The embodiments of test devices (100, 300, 400) will be described in connection with FIGS. 13A to 13D together, to avoid overlapping explanation.

As described above, the test device 400 irradiates light selectively to a certain area of the reactor 20 or controls the irradiated light to selectively pass the reaction chamber 25.

Accordingly, unlike the conventional test device, the test device of exemplary embodiments may prevent degradation of detection ability of the photo detector 150 due to the scattering of light because of the optically transparent property of the reactor.

For example, in a case that the test device of exemplary embodiments takes an image of a low density bio marker, e.g., a cardiovascular disease marker, it may have high detection sensitivity.

Here, the cardiovascular disease marker may be a Cardiac Troponin (Troponin I, TnI; Troponin T, TnT).

The Cardiac Troponin is used as an auxiliary means for diagnosis of acute myocardial infarction (AMI) on the basis of a pain in the chest, a rise in a marker for myocardial damage, electrocardiographic changes, and risk identification of patients with non ST-segment elevation acute coronary syndrome.

When an acute mycardial infarction occurs, the Cardiac TnI and TnT are produced. In the case of the patient with acute mycardial infarction, the TnI and TnT increases in the early stage and reaches a concentration clearly distinguished from a base value, thereby helping to determine the acute mycardial infarction.

FIGS. 13A through 13D show results of comparison between images from which TnI is detected by a conventional test device and images captured with the test device 400 of exemplary embodiments. The reactor 20 was inserted to the test device of exemplary embodiments to examine the cardiovascular marker, TnI.

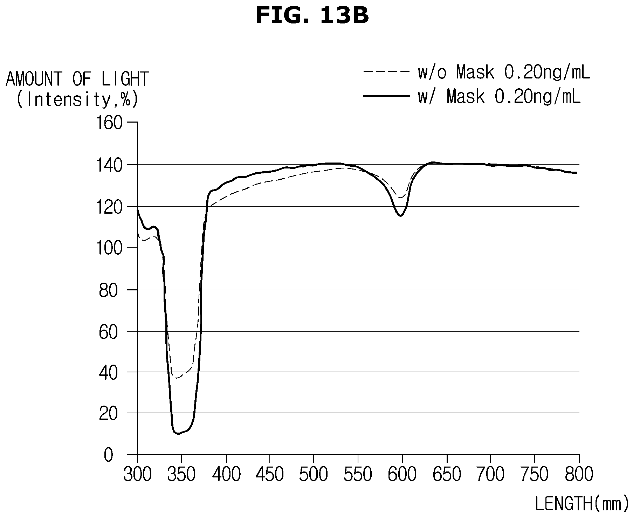

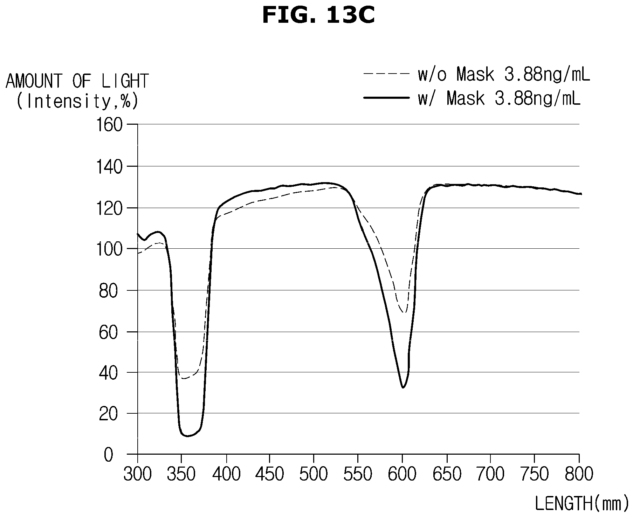

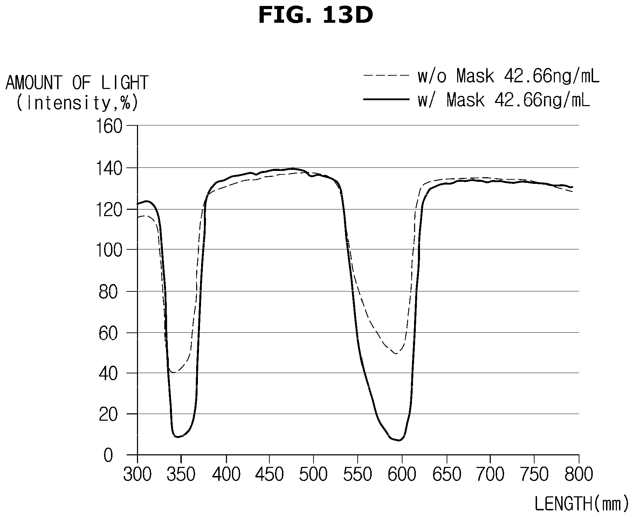

FIGS. 13A, 13B, 13C, and 13D show graphs of comparison with 0.05ng/mL of TnI of the sample, 0.2ng/mL of TnI of the sample, 3.9ng/mL of TnI of the sample, 43ng/mL of TnI of the sample, respectively.

In the graphs, the x-axis represents pixels of the photo detector 150, e.g., the length of the reaction chamber 25 in millimeters (mm). The y-axis represents amounts of light collected by the photo detector 150 or the intensity of light in percentage (%).

In the graphs, dotted plots result from the conventional test device and solid plots result from the test device in accordance with embodiments, described generally by their masking, either by physical masking or selective illumination of light sources.

As mentioned above in connection with FIG. 3, the blood serum centrifuged by rotation of the reactor 20 is absorbed in the reaction chamber 25. The serum moves around inside the reaction chamber 25 while reacting with a reagent contained in the reaction chamber 25. The serum reaches the control line 26 first. The photo detector 150 receives the light that has passed the object for detection in the control line 26 and determines whether the serum normally passes the reaction chamber 25.

For example, FIG. 13A shows results from the photo detector 150 capturing a serum with 0.05ng/mL of TnI while the serum moves around. Specifically, in FIG. 13A, the control line 26 is located within about 350 mm to about 400 mm from the starting point at which the reaction chamber 25 and the channel 24 are connected. It is seen that an amount of light received by the photo detector 150 in the control line 26 is about 15% less than in the conventional technology, to clearly indicate the control condition.

In the conventional test device, the light scattered due to the property of optical transparency of the reactor 20 is collected by the photo detector 150. By preventing this, the photo detector 150 of the test device 100 in accordance with the present disclosure may receive the light that has passed the reaction chamber 25, and the photo detector 150 is able to detect dark light that has penetrated the object for detection.

In the meantime, the serum that has passed the control line 26 keeps moving around the reaction chamber 25 and passes the test line 27. The photo detector 150 may receive the light that has passed the object for detection in the test line 27.

In FIG. 13A, the test line 27 may be located between about 600 mm and about 650 mm. Referring to FIG. 13A, it is seen that dark light is detected by the photo detector 150 in the test line 27 as well.

In an experiment with a changed TnI, it is seen that the test device 100 in accordance with the present disclosure receives dark light that has only passed the first area 25a of the reactor 20. Referring to FIG. 13B to 13D, it is seen that amounts of light collected from the control line 26 and the test line 27 are less than an amount of light received by the conventional test device.

When comparing FIGS. 13A to 13D, it is seen that as the concentration of TnI is heavier, it becomes more difficult for the light irradiated by the light source 130 to pass the object for detection and the photo detector 150 receives less amount of light.

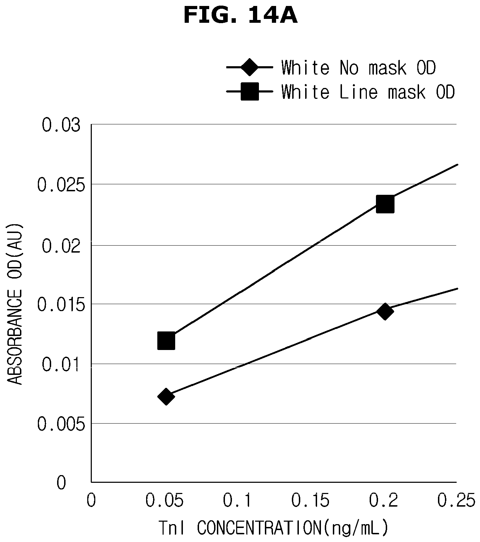

FIGS. 14A to 14C are graphs for explaining effects of a test device according to another exemplary embodiment. The embodiment will be described in connection with FIGS. 14A to 14C together to avoid overlapping explanation.

In FIGS. 14A to 14C, the x-axis represents the concentration of TnI in ng/mL. The y-axis represents absorbance in AU.

The test device 200 in accordance with another exemplary embodiment includes the mask 50 between the light source 130 and the reactor 20.

The mask 50 makes the irradiated light selectively pass the reactor 20, specifically, the reaction chamber 25. This prevents the light scattered in other area than the reaction chamber 25 from being received by the photo detector 150.

To compare effects of the test device 200 in and exemplary embodiment of the disclosure and the conventional test device, the graph represents the conventional test device by a solid plot with diamonds and the test device 200 by a solid plot with squares.

Referring to FIG. 14A, it is seen that the test device 200 has more effective absorbance even with low density TnI of about 0.05 to 0.25 ng/mL. This indicates that sensitivity of detection is improved even for the low concentration.

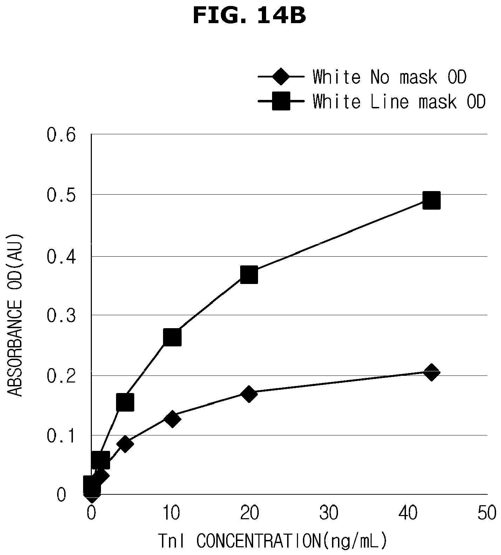

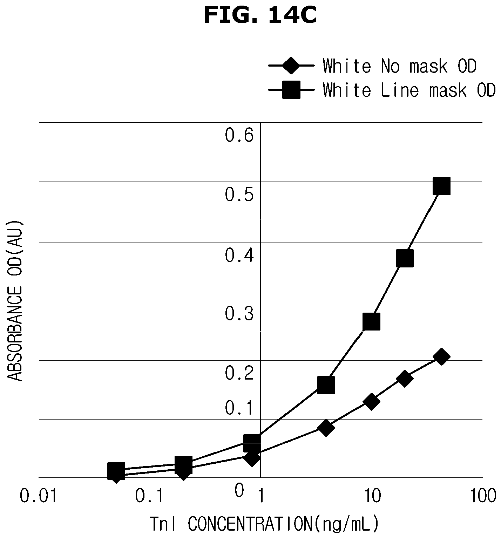

Referring to FIGS. 14B and 14C, the test device 200 detects the object for detection more effectively than in the conventional technology even if the concentration of TnI significantly changes.

For example, in FIG. 14B, the conventional test device has about 0.08 AU of difference in absorbance between when the absorbance is detected at 10 ng//mL of TnI and at 40 ng/mL. In comparison, the test device 200 in an exemplary embodiment has about 0.28 AU of difference in absorbance between when the absorbance is detected at 10 ng//mL of TnI and at 40 ng/mL.

Accordingly, the test device 200 improves a dynamic range because it is able to clearly detect the object for detection in various concentration ranges of a sample.

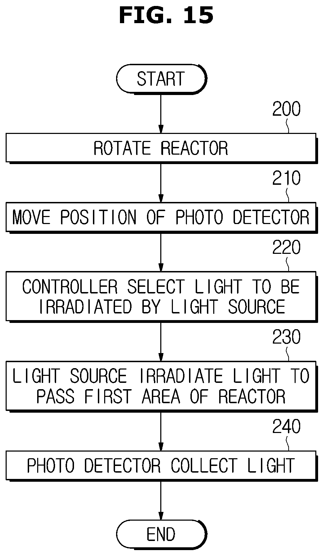

FIG. 15 is a flowchart illustrating operation of a test device, according to an exemplary embodiment. The reference numbers for the test device correspond to that used for FIG. 12. However, the method is not limited thereto.

Referring to FIG. 15, the test device 100 includes the light source 130 with light sources to irradiate light within limited range.

In operation, the reactor 20 is seated on the tray 102 of the test device 100. Subsequently, the tray 102 is inserted into the main body 107 according to an input command from the user.

The reactor 20 is then rotated by power delivered by the driver 140, in 200.

With the rotation of the reactor 20, a sample contained in a sample chamber 23 is moved to the reaction chamber 25 along the channel 24. When the rotation is stopped, the reaction chamber 25 absorbs the sample.

When the rotation of the reactor 20 is stopped, the controller 120 determines a position of the reaction chamber 25 of the reactor 20. This is the case when the controller 120 may control rotation of the reactor 20 such that the reaction chamber 25 is located between the light source 130 and the photo detector 150.

If the reaction chamber 25 is incorrectly located between the light source 130 and the photo detector 150, the controller 120 controls the photo detector 150 to be shifted, in 210.

The controller 120 selects light to be irradiated by the light source 430. For example, if the reaction chamber 25 uses gold nanoparticles, the photo detector 150 may receive the light of green wavelength more effectively than the other wavelengths. Accordingly, the controller 120 may control the light source 430 to irradiate the light of green wavelength, in 220.

The light source 430 irradiates the light to be limited into the first area of the reactor 20, in 230. Specifically, the controller 120 determines the positions of the reaction chamber 25 and the receiver 150, and then controls the light source 430 to irradiate light to be confined to the reaction chamber.

The photo detector 150 collects the light that has passed the object for detection contained in the reaction chamber 25, e.g., sample that has reacted with a reagent, in 240.

As described above in connection with FIGS. 6 to 8, the optically transmissive reactor 20 may scatter the light in other areas than the first area 25a of the reactor 20, e.g., in the second area 25b. If the scattered light is collected by the photo detector 150, the photo detector's 150 ability to detect the light that has passed the object for detection becomes low. In embodiments, a range into which the light source 430 irradiates light is limited, thereby increasing the effect of detection.

FIG. 16 is a flowchart illustrating operation of a test device, according to another exemplary embodiment. The reference numbers for the test device correspond to that used in Fig. 9. However, the method is not limited thereto.

Referring to FIG. 16, the test device 200 further includes the mask 50 to limit the light to selectively pass between the light source for irradiating light and the reactor 20.

In operation, the reactor 20 is seated on the tray 102 of the test device 200. Subsequently, the tray 102 is inserted to the inside of the main body 107 according to an input command from the user.

The reactor 20 is then rotated by power delivered by the driver 140, in 300.

When the rotation of the reactor 20 is stopped, the controller 120 determines a position of the reactor 20, especially the reaction chamber 25.

Based on the position of the reaction chamber 25, the controller 120 adjusts the position of the mask 50 or moves the photo detector 150, in 310.

As described above in connection with FIGS. 9 through 11, the mask 50 may include at least one slit 51 corresponding to the first area 25a of the reactor 20. The slit 51 passes the light irradiated by the light source 130 to the reactor 20. The remaining area of the mask 50 other than the slit 51 reflects the light.

Determining the position of the mask 50, which is performed by the controller 120, includes determining whether the slit 51 of the mask 50 and the first area 25a of the reactor 20 correspond to each other, at 315.

There may be many different ways to make this determination. If the mask slit 51 and the first area 25a do not correspond to each other, the controller 120 may output an indication of whether the position is incorrect to the user through the display 160, at 316.

When correspondence of the mask and reaction area is confirmed, the light source 130 irradiates the light to be limited into the first area of the reactor 20, in 320.

The irradiated light passes the slit 51 of the mask 50 and penetrates the object for detection contained in the reaction chamber 25 of the reactor 20.

The photo detector 150 collects the light that has passed the object for detection contained in the reaction chamber 25, e.g., sample that has reacted with a reagent, in 330.

Accordingly, the test device 200 in accordance with an exemplary embodiment uses the mask 50 to selectively limit the light irradiated by the light source 130, to minimize the photo detector 150 collecting the light that has passed the second area 25b of the reactor 20, thereby increasing the effect and sensitivity of detection.

FIG. 17 is a flowchart illustrating operation of a test device, according to another exemplary embodiment. The reference numbers for the test device correspond to that used in Fig. 12. However, the method is not limited thereto.

Referring to FIG. 17, the test device 400 selectively controls an area into which the light source 430 irradiates light.

In operation, the reactor 20 is seated on the tray 102 of the test device 100. Subsequently, the tray 102 is inserted to the inside of the main body 107 according to an input command from the user.

The reactor 20 is then rotated by power delivered by the driver 140, in 400.

With the rotation of the reactor 20, a sample contained in a sample chamber 23 is moved to the reaction chamber 25 along the channel 24.

When the rotation is stopped, the controller 120 determines the position of the photo detector 150 and an area into which the light source 430 irradiates light, in 410.

Specifically, when the rotation is stopped, the controller 120 determines the position of the photo detector 150 and the area into which the light source 430 irradiates light based on the position of the reaction chamber 25 of the reactor 20.

The controller 120 moves the photo detector 150 based on the determined position, in 420.

Specifically, the controller 120 controls the power deliverer 155 for delivering power from the driver 140 to move the photo detector 150.

The controller 120 controls the light source 430 such that the light irradiated by the light source 430 is confined to pass the first area 25a of the reactor 20, in step 430.

As described above in connection with FIG. 12, the controller 120 selects light sources to be turned on by the light source 430 based on the determined position of the reactor 20. In other words, from among the plurality of light sources included in the light source 430, some light sources are selected to irradiate light only to the first area 25a.

The light selectively irradiated by the light source 430 passes the object for detection and is collected by the photo detector 150, in 440.

This minimizes the photo detector 150 collecting the light scattered in the second area 25b of the reactor 20 and improves the detection ability and sensitivity.

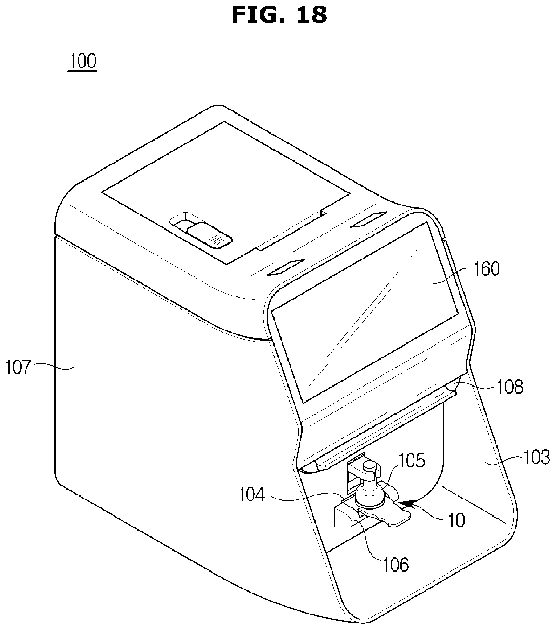

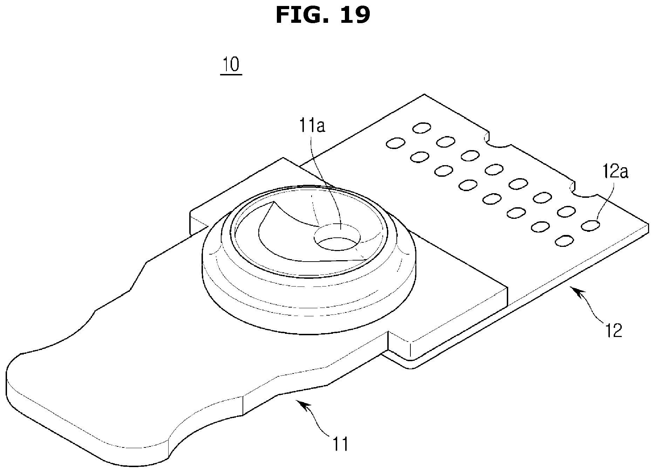

FIG. 18 is another exterior view of a test device, according to another exemplary embodiment. FIG. 19 is an exterior view of a reactor 10 to be inserted into the test device of FIG. 18. The embodiment will be described in connection with FIGS. 18 and 19 together to avoid overlapping explanation.

As described above in connection with FIG. 1, the test device 100 is not necessarily limited to the disc type. The test device 100 may be any in vitro diagnostic device that is able to optically measure a sample contained in the optically transmissive reactor 20.

As an example of the in vitro diagnostic device, FIG. 18 is directed to a test device 500 with a cartridge type reactor 10 inserted thereto.

The test device 500 includes an equipment part 103, which is an area where a reactor 10 is provided. When a door 108 of the equipment part 103 is slid open, the reactor 10 may be inserted in the test device 100. Specifically, part of the reactor 10 may be inserted to an insertion groove 104 formed in the equipment part 103.

The part of the reactor 10 is inserted to the inside of the main body 107 and the remaining part may be exposed outside the test device 100 and supported by a supporter 106. Furthermore, a pressurizer 105 may facilitate movement of the sample into a reaction area by pressing the reactor 10.

After the equipment of the reactor 10 is completed, the door 108 of test device 500 is shut, the test device begins testing operations.

The cartridge type reactor 10 to be inserted into the test device 500 as shown in FIG. 18 may have the exterior as shown in FIG. 19.

Referring to FIG. 19, the reactor 10 in accordance with an exemplary embodiment may include a housing 11, and a platform 12 where a sample and a reagent join and make a reaction.

The housing 11 allows the user to grip the reactor 10 while supporting the platform 12. The platform 12 may be combined with the housing 11 in such a way as to be connected to the bottom of the housing 11, or to be inserted into a groove formed in the housing 11.

The housing 11 is easy to be molded, and may be formed of a chemically and biologically inactive material. For example, various materials including plastic materials, such as acryl, e.g., polymethylmethacrylate (PMMA), polysiloxane, e.g., polydimethylsiloxane (PDMS), polycarbonate (PC), linear low density polyethylene (LLDPE), low density polyethylene (LDPE), medium density polyethylene (MDPE), high density polyethylene (HDPE), polyvinyl alcohol, very low density polyethylene (VLDPE), polypropylene (PP), acrylonitrile butadiene styrene (ABS), cycloolefin copolymer (COC), etc., glass, mica, silica, semiconductor wafer, etc., may be used as the material for the housing 11.

An inlet hole 11a, through which the sample comes in, is formed in the housing 11. The user may use a tool, e.g., a pipette or a syringe to drop a sample into the inlet hole 11a for tests.

A plurality of chambers 12a are formed on the platform 12, each chamber 12a containing a reagent. For example, the reagent may be contained in a manner of being applied within the chamber 12a and then dried. The sample coming in through the inlet hole 11a reaches the chamber 12a through a channel (not shown) that connects the inlet hole 11a and the chamber 12a and reacts with the reagent contained in the chamber 12a. Turning back to FIG. 18, part of the reactor 10 is inserted to the insertion groove 104 of the test device 500. Since a reaction between the reagent and the sample occurs in the chamber 12a, the platform 12 may be inserted to the groove 104 and the pressurizer 105 presses the inlet hole 11a to facilitate the introduction of the sample.

Although not shown, the platform 12 may have a structure in which three plates are joined. The three plates may be divided into top, middle, and bottom plates and the top and bottom plates may be printed with light shielding ink to protect the sample that moves into the chamber 12a from being affected by light.

The top and bottom plates are made of films. The films used to form the top and bottom plates may be one selected from among polyethylene films, such as VLDPE, LLDPE, LDPE, MDPE, HDPE, etc., PVC films, PVA films, polystyrene films, and PET films.

The middle plate may be formed of a porous sheet, such as cellulose to serve as a vent in itself, and the porous sheet may be made of a hydrophobic material or be subjected to hydrophobic process in order to influence the movement of the sample.

The platform 12 with the tri-layered structure may have a hole formed in the top and middle plates to form the inlet hole 11a and a part corresponding to the chambers 12a of the top and bottom plates, which may be processed to be transparent.

The test device 500 may also control the light source 130 or the mask 50 to irradiate light in a confined or limited manner to the optically transmissive chambers 12a of the top and bottom plates, thereby minimizing the scattering of light.

A thin channel may be formed in the middle plate, and the sample coming in through the inlet hole 11a may be moved to the chambers 12a by capillary force of the channel.

The chambers 12a may contain reagents, and the light source 130 may limitedly irradiate light to correspond to the size and positions of the chambers 12a. In the case of an example of the reactor 10, the light source 130 may irradiate light in the form of a point light source.

FIG. 20 is another example of an exterior view of a reactor.

FIG. 20 shows the disc type reactor 20, which may further include identification information 30 in addition to what are included in the reactor 20 as described above in connection with FIG. 2.

The identification information 30 may be formed as a Quick Response (QR) code as shown in FIG. 20 and attached to the front of the reactor 20. Alternatively the identification information may include at least one of a bar code, text data, a data matrix, a recognition pattern, Near Field Communication (NFC), and Radio Frequency Identification (RFID) and attached to the reactor 20.

Once the reactor 20 is inserted to the test device 100, the test device 100 may recognize the identification information 30 attached to the reactor 20 to obtain data about a proper amount of light, temperature, sample, etc., of the reactor 20 for a predetermined test.

For example, if the reaction chamber 25 of the reactor 20 uses a gold nanoparticles, the light source may irradiate the light of green wavelength based on the identification information 30.

The identification information 30 may be included in any type of reactor besides the reactor 20 shown in FIG. 20, without being limited thereto.

According to exemplary embodiments, a test device, test system, and control method of the test device defines a light irradiating area in a reactor, thereby preventing a decrease in magnitude of a detected signal that might occur due to scattering of light that has penetrated areas of the reactor, other than an area containing an object for detection, thereby improving a dynamic range.

## Claims

1. A test device comprising:
a light source configured to emit light;
a reactor including at least one first area configured to contain an object for detection; and
a photo detector configured to receive the light that has been emitted from the light source and has passed through the reactor that contains the object for detection,
wherein the light source is configured to limit the light emitted to the first area of the reactor.

2. The test device of claim 1, further comprising a controller configured to control the light source to emit the light to be limited into the first area that contains the object for detection of the reactor.

3. The test device of claim 1, wherein the first area has a form of a strip.

4. The test device of claim 1, wherein the light source comprises a linear light source configured to emit a line of the light having a width less than a width of the first area of the reactor.

5. The test device of claim 2, wherein the controller limits a width of the light emission to be less than a width of the first area of the reactor.

6. The test device of claim 2, wherein the controller is configured to control the photo detector to be moved to a position to receive the light that has passed through the first area.

7. The test device of claim 6, further comprising a driver configured to rotate the reactor,
wherein the controller is configured to control the driver to rotate the reactor and to position the reactor such that the light emitted by the light source passes through the first area.

8. A test system comprising:
a light source configured to emit light;
a photo detector configured to receive the light that has been emitted by the light source and has passed through an object for detection;
a reactor including at least one first area configured to contain the object for detection; and
a mask located between the light source and the reactor to prevent the light emitted by the light source from irradiating at least one second area in the reactor.

9. The test system of claim 8, wherein the mask comprises a slit configured to allow the light emitted by the light source to limitedly pass to the first area of the reactor.

10. The test system of claim 8, wherein the mask comprises a slit having a width less than the width of the first area of the reactor.

11. The test system of claim 8, wherein the mask is formed with a slit, and the at least one first area and the slit have the form of a strip.

12. The test system of claim 8, wherein the light source comprises a back light unit including at least one of a point light source, a linear light source, and an area light source.

13. The test system of claim 12, further comprising a driver configured to rotate the reactor,
wherein the driver is configured to rotate the reactor and to move the photo detector based on positions of a slit formed in the mask and the first area of the reactor.

14. A test device comprising:
a light source configured to emit light;
a photo detector configured to receive the light that has been emitted by the light source and has passed through a reactor that contains an object for detection; and
a controller configured to control the light source to emit the light to be limited into a first area that contains the object for detection of the reactor,
wherein the light source comprises:
a back light unit configured to include at least one of a point light source, a linear light source, and an area light source; and
a display panel configured to control an area to which the back light unit emits the light.

15. The test device of claim 14,
wherein the reactor comprises identification information having data about the object for detection, and
wherein the controller is configured to control the light source to emit the light onto the identification information.
